# EUROPEAN PATENT APPLICATION

(11) **EP 1 086 692 A2**
(43) Date of publication of application: **28.03.2001**
(21) Application number: 00305611.6
(22) Date of filing: 03.07.2000
(51) Int. Cl.: A61K 31/40, A61K 31/35, A61K 31/135, A61P 19/10, A61P 9/00, A61P 35/00

(54) **Estrogen agonists and antagonists for multiple indications**

(30) Priority: 28.07.1999 US 146072 P; 28.07.1999 US 146075 P
(71) Applicant: Pfizer Products Inc., Groton, Connecticut 06340 (US)
(72) Inventor: Yu, Li Julia, Groton, Connecticut 06340 (US)
(74) Representative: Wood, David John

(57) **Abstract**

The use of an estrogen agonist for the manufacture of a medicament for the treatment or prevention of osteoporosis, cardiovascular disease or breast cancer in a human patient under treatment with warfarin or propranolol without interfering with the action of said warfarin or propranolol.

## Description

This invention relates to estrogen agonists and their pharmaceutical uses.

### BACKGROUND OF THE INVENTION

The value of naturally occurring estrogens and synthetic compositions demonstrating "estrogenic" activity has been in their medical and therapeutic uses. A traditional listing of the therapeutic applications for estrogens alone or in combination with other active agents includes: oral contraception; relief for the symptoms of menopause; prevention of threatened or habitual abortion; relief of dysmenorrhea; relief of dysfunctional uterine bleeding; an aid in ovarian development; treatment of acne; diminution of excessive growth of body hair in women (hirsutism); the prevention of cardiovascular disease; treatment of osteoporosis; treatment of prostatic carcinoma; and suppression of post-partum lactation [Goodman and Gilman, The Pharmacological Basis Of Therapeutics (Seventh Edition) Macmillan Publishing Company, 1985, pages 1421-1423]. Accordingly, there has been increasing interest in finding newly synthesized compositions and new uses for previously known compounds which are demonstrably estrogenic, that is, able to mimic the action of estrogen in estrogen responsive tissue.

From the viewpoint of pharmacologists interested in developing new drugs useful for the treatment of human diseases and specific pathological conditions, it is most important to procure compounds with some demonstrable estrogen-like function but which are devoid of proliferative side-effects. Exemplifying this latter view, osteoporosis, a disease in which bone becomes increasingly more fragile, is greatly ameliorated by the use of fully active estrogens; however, due to the recognized increased risk of uterine cancer in patients chronically treated with active estrogens, it is not clinically advisable to treat osteoporosis in intact women with fully active estrogens for prolonged periods. Accordingly estrogen agonists are the primary interest and focus.

Osteoporosis is a systemic skeletal disease, characterized by low bone mass and deterioration of bone tissue, with a consequent increase in bone fragility and susceptibility to fracture. In the U.S., the condition affects more that 25 million people and causes more than 1.3 million fractures each year, including 500,000 spine, 250,000 hip and 240,000 wrist fractures annually. These cost the nation over $10 billion. Hip fractures are the most serious, with 5-20% of patients dying within one year, and over 50% of survivors being incapacitated.

The elderly are at greatest risk of osteoporosis, and the problem is therefore predicted to increase significantly with the aging of the population. Worldwide fracture incidence is forecast to increase three-fold over the next 60 years, and one study estimates that there will be 4.5 million hip fractures worldwide in 2050.

Women are at greater risk of osteoporosis than men. Women experience a sharp acceleration of bone loss during the five years following menopause. Other factors that increase the risk include smoking, alcohol abuse, a sedentary lifestyle and low calcium intake.

Estrogen is the agent of choice in preventing osteoporosis or post menopausal bone loss in women; it is the only treatment which unequivocally reduces fractures. However, estrogen stimulates the uterus and is associated with an increased risk of endometrial cancer. Although the risk of endometrial cancer is thought to be reduced by a concurrent use of a progestogen, there is still concern about possible increased risk of breast cancer with the use of estrogen.

Three classes of agents are currently approved in the U.S. for the prevention of osteoporosis in women, calcitonin, estrogen (with or without opposing progestins) and bisphosphonate. Calcitonin has been shown to prevent postmenopausal bone loss and offer analgestic benefits. However, calcitonin's use is limited by high cost, parenteral delivery, and tachyphylaxis. Bisphosphonate has recently been approved for the treatment of osteoporosis and shows good efficacy in reducing of fracture, but does not have the beneficial effects on cardiovascular system or in breast cancer. Clinically, estrogen replacement therapy in postmenopausal women is efficacious in decreasing bone turnover, reducing the incidence of vertebral and hip fractures by about 50%, decreasing the occurrence of ischemic heart disease by up to 50% and in relieving menopausal symptoms.

One third of hip fractures occurs in man. However estrogen is not suitable for use in elderly man. A new therapy for male osteoporosis is a great medical need.

Black, et al. in EP 0605193A1 report that estrogen, particularly when taken orally, lowers plasma levels of LDL and raises those of the beneficial high density lipoproteins (HDL's). Long-term estrogen therapy, however, has been implicated in a variety of disorders, including an increase in the risk of uterine cancer and possibly breast cancer, causing many women to avoid this treatment. Recently suggested therapeutic regimens, which seek to lessen the cancer risk, such as administering combinations of progestogen and estrogen, cause the patient to experience unacceptable bleeding. Furthermore, combining progesterone with estrogen seems to blunt the serum cholesterol lowering effects of estrogen. The significant undesirable effects associated with estrogen therapy support the need to develop alternative therapies for hypercholesterolemia that have the desirable effect on serum LDL but do not cause undesirable effects.

There is a need for improved estrogen agonists which exert selective effects on different tissues in the body.

Estrogen agonists have multiple beneficial effects including preventative or pallative effects on osteoporosis, breast cancer and cardiovascular disease. These compounds are therefor beneficial for and are prescribed for long terms of treatment to healthy individuals. Under these circumstances it is desirable that unwanted or harmful side effects be avoided. In particular interference with other necessary medications must be avoided.

We have now found that lasofoxifene and droloxifene and their pharmaceutically acceptable salts do not interfere with the binding of warfarin and propranolol to human plasma protein thus showing that lasofoxifene and droloxifene will not have any undesired interaction with these two drugs related to protein binding displacement from plasma proteins.

### Summary of the Invention

This invention provides a method for treating and preventing osteoporosis, cardiovascular disease and breast cancer without displacing warfarin or propranolol from plasma proteins in a human patient under treatment with warfarin or propranolol, which comprises treating said patient with a pharmaceutically effective amount of an estrogen agonist.

In another aspect this invention provides a method for treating and preventing osteoporosis, cardiovascular disease and breast cancer without interfering with the action of warfarin or propranolol in a human patient which comprises treating said patient with a pharmaceutically effective amount of lasofoxifene or a pharmaceutically acceptable salt thereof.

In another aspect this invention provides a method for treating and preventing osteoporosis, cardiovascular disease and breast cancer without interfering with the action of warfarin or propranolol in a human patient which comprises treating said patient with a pharmaceutically effective amount of droloxifene or a pharmaceutically acceptable salt thereof.

### Detailed Description of the Invention

Droloxifene and its pharmaceutically acceptable salts are known to be effective for the treatment and prevention of osteoporosis (U.S. Patent No. 5,254,594); cardiovascular disease (U.S. Patent No. 5,902,830); and breast cancer (I. Kawamura, et al, Japan J. Pharmacol 57, 215-224 (1991).

The preparation of lasofoxifene ((-) cis-6-phenyl-5[4-(2-pyrrolitin-1-yl-ehtoxy)-phenyl]-5,6,7,8-tetrahydronaphthalene-2-ol) and pharmaceutically acceptable salts thereof is described in U.S. Pat. No. 5,552,412 which is incorporated herein by reference.

The use of lasofoxifene and pharmaceutically acceptable salts thereof for the treatment of osteoporosis, breast cancer, cardiovascular disease and hypercholesterolemia is also described in U.S. Patent No. 5,552,412.

As used in this application, "Cardiovascular disease" means hypercholesterolemia and atherosclerosis. "Treating" means curing, alleviating the symptoms of or preventing or reducing the incidence of the onset of a disease or condition.

The remedies for the prostatic, cardiovascular and bone diseases of this invention comprise, as active ingredient, lasofoxifene or droloxifene or a salt thereof. The pharmaceutically acceptable salts of lasofoxifene and droloxifene are salts of non-toxic type commonly used, such as salts with organic acids (e.g., formic, acetic, trifluoroacetic, citric, maleic, tartaric, methanesulfonic, benzenesulfonic or toluenesulfonic acids), inorganic acids (e.g. hydrochloric, hydrobromic, sulfuric or phosphoric acids), and amino acids (e.g., aspartic or glutamic acids). These salts may be prepared by the methods known to chemists of ordinary skill.

The remedies for prostatic, cardiovascular and bone diseases, of this invention may be administered to animals including humans orally or parenterally in the conventional form of preparations, such as capsules, microcapsules, tablets, granules, powder, troches, pills, suppositories, injections, suspensions and syrups.

The remedies for prostatic and cardiovascular and bone diseases of this invention can be prepared by the methods commonly employed using conventional, organic or inorganic additives, such as an excipient (e.g., sucrose, starch, mannitol, sorbitol, lactose, glucose, cellulose, talc, calcium phosphate or calcium carbonate), a binder (e.g., cellulose, methylcellulose, hydroxymethylcellulose, polypropylpyrrolidone, polyvinylprrolidone, gelatin, gum arabic, polyethyleneglycol, sucrose or starch), a disintegrator (e.g., starch, carboxymethylcellulose, hydroxypropylstarch, low substituted hydroxypropylcellulose, sodium bicarbonate, calcium phosphate or calcium citrate), a lubricant (e.g., magnesium stearate, light anhydrous silicic acid, talc or sodium lauryl sulfate), a flavoring agent (e.g., citric acid, menthol, glycine or orange powder), a preservative (e.g., sodium benzoate, sodium bisulfite, methylparaben or propylparaben), a stabilizer (e.g., citric acid, sodium citrate or acetic acid), a suspending agent (e.g., methylcellulose, polyvinylpyrrolidone or aluminum stearate), a dispersing agent (e.g., hydroxypropylmethylcellulose), a diluent (e.g., water), and base wax (e.g., cocoa butter, white petrolatum or polyethylene glycol). The amount of the active ingredient in the medical composition may be at a level that will exercise the desired therapeutical effect; for example, about 1 mg to 100 mg in unit dosage for both oral and parenteral administration.

The active ingredient may be usually administered once to four times a day with a unit dosage of 0.1 mg to 100 mg in human patients, but the above dosage may be properly varied depending on the age, body weight and medical condition of the patient and the type of administration. One dose per week is preferred.

As used in this application, "prevention" means avoidance or reduction of the incidence of the appearance of occult disease.

The remedies for the prevention of breast cancer of this invention comprise, as active ingredient, lasofoxifene or droloxifene or a salt thereof. The pharmaceutically acceptable salts of lasofoxifene are salts of non-toxic types commonly used, such as salts with organic acids (e.g., formic, acetic, trifluoroacetic, citric, maleic, tartaric, methanesulfonic, benzenesulfonic or toluenesulfonic acids), inorganic acids (e.g. hydrochloric, hydrobromic, sulfuric or phosphoric acids), and amino acids (e.g., aspartic or glutamic acids). These salts may be prepared by the methods known to chemists of ordinary skill.

The remedies for prevention of breast cancer of this invention may be administered to animals including humans orally or parenterally in the conventional form of preparations, such as capsules, microcapsules, tablets, granules, powder, troches, pills, suppositories, injections, suspensions and syrups.

The remedies for prevention of breast cancer of this invention can be prepared by the methods commonly employed using conventional, organic or inorganic additives, such as an excipient (e.g., sucrose, starch, mannitol, sorbitol, lactose, glucose, cellulose, talc, calcium phosphate or calcium carbonate), a binder (e.g., cellulose, methylcellulose, hydroxymethylcellulose, polypropylpyrrolidone, polyvinylprrolidone, gelatin, gum arabic, polyethyleneglycol, sucrose or starch), a disintegrator (e.g., starch, carboxymethylcellulose, hydroxypropylstarch, low substituted hydroxypropylcellulose, sodium bicarbonate, calcium phosphate or calcium citrate), a lubricant (e.g., magnesium stearate, light anhydrous silicic acid, talc or sodium lauryl sulfate), a flavoring agent (e.g., citric acid, menthol, glycine or orange powder), a preservative (e.g., sodium benzoate, sodium bisulfite, methylparaben or propylparaben), a stabilizer (e.g., citric acid, sodium citrate or acetic acid), a suspending agent (e.g., methylcellulose, polyvinylpyrrolidone or aluminum stearate), a dispersing agent (e.g., hydroxypropylmethylcellulose), a diluent (e.g., water), and base wax (e.g., cocoa butter, white petrolatum or polyethylene glycol). The amount of the active ingredient in the medical composition may be at a level that will exercise the desired therapeutical effect; for example, about 1 mg to 100 mg in unit dosage for both oral and parenteral administration.

The active ingredient may be usually administered once to four times a day with a unit dosage of 0.25 mg to 100 mg in human patients, but the above dosage may be property varied depending on the age, body weight and medical condition of the patient and the type of administration. One dose per day is preferred.

The exact mechanism of an antiestrogen in treating or preventing mammary cancer is not known. It is believed, however, that the antiestrogen, circulating in the body, competes with endogenous estrogens for estrogen receptor sites in the cancer. To be effective, the antiestrogen must find and bind to receptor sites, and prevent estradiol from occupying them.

Accordingly, a most important embodiment of the present invention is a method of preventing mammary cancers which comprises administering lasofoxifene in an effective amount to a patient at risk of such a cancer.

Lasofoxifene and droloxifene are selective estrogen receptor modulators (SERMs), which are useful for the prevention of postmenopausal bone loss, the reduction of cardiovascular disease, and prevention of breast cancer. However, both are highly bound to human plasma proteins (99.997%). To assess the potential drug-drug interactions caused by competitive protein binding, the binding of warfarin or propranolol to human plasma proteins was investigated in the absence of presence of lasofoxifene and droloxifene. As described in Example 1 the protein binding of warfarin or propranolol in human plasma is not altered in the presence of lasofoxifene or droloxifene.

### EXAMPLES

### EXAMPLE 1

### Effect of Droloxifene and Lasofoxifene on the Binding of Warfarin and Propranolol to Human Plasma Proteins

Droloxifene and lasofoxifene, selective estrogen receptor modulators (SERM), are useful for the prevention of postmenopausal bone loss, the reduction of cardiovascular disease, and prevention of breast cancer. However, they are highly bound to human plasma proteins (99.997 and 99.94% for droloxifene and lasofoxifene, respectively). To assess the potential drug-drug interactions caused by competitive protein binding, the binding of warfarin or propranolol to human plasma proteins was investigate in the absence and presence of droloxifene and lasofoxifene. The protein binding of warfarin or propranolol was determined using equilibrium dialysis. The effect of droloxifene or lasofoxifene on plasma protein binding was assessed at wart arm concentrations of 2 and 10 µg/ml and propranolol concentrations of 50 and 500 ng/ml. The concentrations of droloxifene and lasofoxifene used were 200 and 50 ng/ml respectively. Traditionally, the determination of protein binding of warfarin or propranolol has been limited by the purity of commercially available radiolabelled compounds (typically 97-99%). HPLC-MS assays for both warfarin and propranolol allowed the use of non-labelled compounds and provided very accurate determinations of protein binding. The mean binding of warfarin to human plasma proteins at concentrations of 2 and 10 µg/ml was 99.35% and 99.22% when dialyzed independently, 99.28% and 99.00% in the presence of 200ng/ml droloxifene and 99.31% and 99.15% in the presence of 50 ng/ml lasofoxifene, respectively. The mean binding of propalolol to human plasma proteins at concentrations of 50 and 500 ng/ml was 785 and 76% when dialyzed independently, 74% and 76% in the presence of 200 ng/ml droloxifene and 72% and 80% in the presence of 50 ng/ml lasofoxifene, respectively. These studies show that the protein binding of warfarin or propranolol in human plasma will not be altered in the presence of either droloxifene or lasofoxifene.

### EXAMPLE 2

Twelve thousand women are selected for the clinical study. The women are at enhanced risk of breast cancer because of age (over 60 years) or a family history of breast cancer, but otherwise are in good general health. The study has a placebo control group, i.e., the women are divided into two groups, one of which receives a salt of lasofoxifene as the active agent and the other receives a placebo. Women in the test group receive between 20 - 80 mg of the drug per day by the oral route. They continue this therapy for three years. Each patient is examined for breast cancer by mammography every twelve months. A smaller number of newly developed breast cancer in the group receiving lasofoxifene indicates that lasofoxifene prevents breast cancer.

## Claims

1. The use of an estrogen agonist for the manufacture of a medicament for the treatment or prevention of osteoporosis, cardiovascular disease or breast cancer in a human patient under treatment with warfarin or propranolol without interfering with the action of said warfarin or propranolol.

2. The use of claim 1 wherein the estrogen agonist is droloxifene or a pharmaceutically acceptable salt thereof.

3. The use of claim 1 wherein the estrogen agonist is lasofoxifene, or a pharmaceutically acceptable salt thereof.
